# EUROPÄISCHE PATENTSCHRIFT

(11) **EP 2 049 008 B1**
(45) Veröffentlichungstag und Bekanntmachung des Hinweises auf die Patenterteilung: **17.02.2010**
(21) Anmeldenummer: 07788110.0
(22) Anmeldetag: 01.08.2007
(51) Int. Cl.: G01N 33/487

(54) **VERPACKUNG ENTHALTEND EIN MIKROFLUIDIKELEMENT MIT HYDROPHILER OBERFLÄCHENBESCHICHTUNG**
PACKAGING CONTAINING A MICROFLUIDIC ELEMENT HAVING A HYDROPHILIC SURFACE COATING
EMBALLAGE CONTENANT UN ÉLÉMENT MICROFLUIDIQUE À REVÊTEMENT DE SURFACE HYDROPHILE

(30) Priorität: 02.08.2006 EP 06016067
(43) Veröffentlichungstag der Anmeldung: 22.04.2009
(73) Patentinhaber: F. Hoffmann-La Roche AG, 4070 Basel (CH); Roche Diagnostics GmbH, 68305 Mannheim (DE)
(72) Erfinder: NIEDERBERGER, Brigitte, 8800 Thalwil (CH); CALASSO, Irio Giuseppe,, 6415 Arth (CH); GLAUSER, Michael, 6341 Rotkreuz (CH)
(74) Vertreter: Pfiz, Thomas
(86) Internationale Anmeldenummer: PCT/EP2007/057939
(87) Internationale Veröffentlichungsnummer: WO 2008/015227

(56) Entgegenhaltungen:
- EP-A- 1 360 935
- WO-A-2006/021361
- US-A- 3 532 412
- US-A- 5 766 473
- US-A- 5 934 494
- US-A1- 2003 087 292
- US-A1- 2004 028 931

## Beschreibung

Die Erfindung betrifft eine Verpackung enthaltend ein Mikrofluidikelement mit hydrophiler Oberfläche.

Hydrophile Oberflächen, insbesondere Oberflächen mit einer hydrophilen Beschichtung, finden besondere Anwendung bei Mikrofluidikelementen, d.h. Mikrostrukturen, durch die eine Flüssigkeit fließen soll. Derartige Mikrofluidikelemente werden speziell in Analysesystemen für Körperflüssigkeiten, bspw. in Blutzuckermessgeräten, mit denen Diabetiker selbst ihren Blutzuckerspiegel kontrollieren können, verwendet. Das mikrofluidische Testelement kann in diesem Fall ein Stechorgan umfassen, welches mit einem Mikrokanal zum kapillaren Transport der Körperflüssigkeit versehen oder verbunden ist. Solche Mikronadeln bzw. Microsampler, wie sie beispielsweise aus der WO 2006/021361 hervorgehen, sind in der Regel als Einwegteil vorgesehen.

Der Mikrokanal und ggf. das Stechorgan sollte aus einem biokompatiblen Material gefertigt sein, das sowohl mechanisch beanspruchbar als auch einfach sterilisierbar sein muss. Besonders geeignet ist hierfür Chirurgenstahl, der allerdings zu wenig hydrophil ist, um einen kapillaren Transport von wässrigen Körperflüssigkeiten durch den Mikrokanal zu gestatten. Aus diesem Grund ist es vorteilhaft, derartige Mikrofluidikelemente mit einer hydrophilen Oberflächenbeschichtung auszustatten. Die Oberflächenbeschichtung sollte außerdem biokompatibel und sterilisierbar sein und die Befüllung des Mikrokanals innerhalb einer sehr kurzen Zeit gestatten.

Schließlich ist eine ausreichende Langzeitstabilität der Oberflächenbeschichtung erwünscht. Dies stellt ein Problem dar, weil hydrophile Beschichtungen in der Regel hochenergetische Oberflächen aufweisen. Dies ist thermodynamisch ungünstig, weil die Oberfläche bestrebt ist, ihre hohe Energie zu reduzieren, indem die Hydrophilie reduziert wird. Dies geschieht bspw. durch die Adsorption von apolaren Gasmolekülen. Verpackungsmaterialien, insbesondere polymere Verpackungsmaterialien können jedoch einen beträchtlichen Anteil an apolaren Gasen enthalten, welche mit der Zeit aus dem Verpackungsmaterial austreten. Außerdem wird unabhängig hiervon auch immer Luft in eine Verpackung eingeschlossen. Es ist daher erwünscht, den Anteil unerwünschter Gase in einer Verpackung zu reduzieren.

Für die Adsorption unerwünschter Gase in Verpackungen werden im Allgemeinen Trockenmittel wie Aktivkohle, Silikagele und Molekularsiebe eingesetzt (vgl. EP 0 951 939 A2). Die Verwendung dieser Materialien kann jedoch bei Gegenständen mit hydrophiler Oberflächenbeschichtung für medizinische Zwecke, wie Mikronadeln oder Lanzetten, problematisch sein. Außerdem ist unklar, inwieweit die Menge der besonders unerwünschten apolaren Gase reduziert wird.

Die Aufgabe der vorliegenden Erfindung besteht somit darin, eine Verpackung enthaltend ein Mikrofluidikelement mit einer hydrophilen Oberfläche bereitzustellen, in welcher die Hydrophilie der Oberfläche während einer möglichst langen Lagerdauer im Wesentlichen erhalten bleibt und die einfach herzustellen und zu verwenden ist. Speziell soll dies den Einsatz von disposiblen mikrofluidischen Testelementen in Analysesystemen verbessern.

Zur Lösung dieser Aufgabe wird die im unabhängigen Anspruch 1 angegebene Merkmalskombination vorgeschlagen. Vorteilhafte Ausgestaltungen und Weiterbildungen der Erfindung ergeben sich aus den abhängigen Ansprüchen.

Demnach wird vorgeschlagen, dass innerhalb der Verpackung mindestens eine lose Abdeckung und/oder mindestens eine adsorbierende Oberfläche vorgesehen ist, deren Affinität für apolare Gase gleich groß oder größer ist als diejenige der hydrophilen Oberfläche.

Es hat sich überraschenderweise herausgestellt, dass bereits eine lose, d.h. nicht verklebte oder sonst wie befestigte Abdeckung, bspw. ein Deckplättchen oder ein Deckvlies, als Abschirmung genügt, um den Zutritt apolarer Gase zu der hydrophilen Oberfläche des verpackten Gegenstandes in einem solchen Ausmaß zu erschweren, dass die Hydrophilie der Oberfläche auch über lange Lagerzeiträume zuverlässig stabil bleibt. Es ist lediglich darauf zu achten, dass die lose Abdeckung selbst keine apolaren Gase freisetzt.

Das gleiche Ziel wird erreicht, wenn innerhalb der Verpackung eine zusätzliche adsorbierende Oberfläche vorhanden ist, die selbst apolare Gase mindestens gleich gut adsorbiert wie die hydrophile Oberfläche des verpackten Gegenstands.

Mit der erfindungsgemäßen Verpackung kann die Adsorption apolarer Gase auf die hydrophile Oberfläche auf besonders einfache und wirkungsvolle Weise reduziert werden, so dass die erfindungsgemäße Verpackung insbesondere für medizinische Massenprodukte gut geeignet ist, ohne die Kosten übermäßig zu erhöhen.

Gemäß einer vorteilhaften Ausführung kann die mindestens eine adsorbierende Oberfläche in Form mindestens eines mit dem Gegenstand verpackten Adsorberelements mit adsorbierender Oberfläche ausgebildet sein. Ein solches Adsorberelement, bspw. in Form eines Plättchens oder Vlieses, kann beim Verpacken des Gegenstandes mit hydrophiler Oberfläche auf einfache Weise maschinell hinzugefügt werden.

Das mindestens eine Adsorberelement kann insbesondere als Abdeckung für die hydrophile Oberfläche selbst ausgebildet sein, so dass der Zutritt apolarer Gase zur hydrophilen Oberfläche zusätzlich erschwert wird.

In einer anderen Ausgestaltung kann das mindestens eine Adsorberelement eine Adsorberschicht in Form einer hydrophilen Beschichtung aufweisen. Dabei sollte die Hydrophilie, das heißt die Oberflächenenergie der Adsorberschicht gleich oder größer sein als die Hydrophilie, das heißt die Oberflächenenergie der Oberfläche des verpackten Gegenstands, um eine effektive Adsorption der apolaren Gase an die Adsorberschicht sicherzustellen. Die hydrophile Beschichtung kann aus dem gleichen Material bestehen wie die hydrophile Oberfläche des verpackten Gegenstands, so dass die Adsorberschicht automatisch biokompatibel und sterilisierbar ist.

Eine andere Ausgestaltung der erfindungsgemäßen Adsorberschicht besteht darin, dass die Innenseite der Verpackung zumindest teilweise mit einer hydrophilen Beschichtung versehen ist, deren Hydrophilie bzw. Oberflächenenergie gleich oder größer ist als die Hydrophilie bzw. Oberflächenenergie der Oberfläche des verpackten Gegenstands, um eine effektive Adsorption der apolaren Gase an die Adsorberschicht sicherzustellen. Auch in diesem Fall kann die hydrophile Beschichtung aus dem gleichen Material bestehen wie die hydrophile Oberflächenbeschichtung des verpackten Gegenstands, so dass die Adsorberschicht automatisch biokompatibel und sterilisierbar ist.
Es sind zahlreiche Materialien für hydrophile Beschichtungen allgemein bekannt, wie bspw. Lecithin oder Dextransulfat. Besonders geeignete Materialien, welche in einer hydrophilen Beschichtung enthalten sein können oder aus denen eine hydrophile Beschichtung bestehen kann, sind Polyacrylsäuren und Polyacrylate. Diese sind biokompatibel und sterilisierbar und daher insbesondere für die Beschichtung von Verpackungen im medizinischen Bereich, bspw. für Mikrofluidiksysteme mit hydrophiler Oberflächenbeschichtung, wie zum Beispiel Mikronadeln für tragbare Blutzuckermessgeräte, besonders gut geeignet.

Die Erfindung betrifft die Einheit aus Verpackung mit Inhalt, d.h. eine Verpackung enthaltend ein Mikrofluidikelement mit einer hydrophilen Oberfläche zur Aufnahme einer Körperflüssigkeit und mindestens eine die hydrophile Oberfläche überdeckende lose Abdeckung und/oder mindestens eine adsorbierende Oberfläche, deren Affinität für apolare Gase gleich groß oder größer ist als diejenige der hydrophilen Oberfläche.

Im Folgenden wird die Erfindung anhand des in der Zeichnung schematisch dargestellten Ausführungsbeispiels näher erläutert. Es zeigen
- Fig. 1: eine Verpackung für Mikrofluidikelemente in einer schaubildlichen Darstellung;
- Fig. 2: eine schematische Darstellung eines Testplättchens mit definier- ten Kapillarbereichen;
- Fig. 3: ein Säulendiagramm zum Befüllverhalten ohne Schutz der hydrophilen Oberfläche;
- Fig. 4: ein Säulendiagramm zum Befüllverhalten mit Schutz der hydrophi- len Oberfläche.

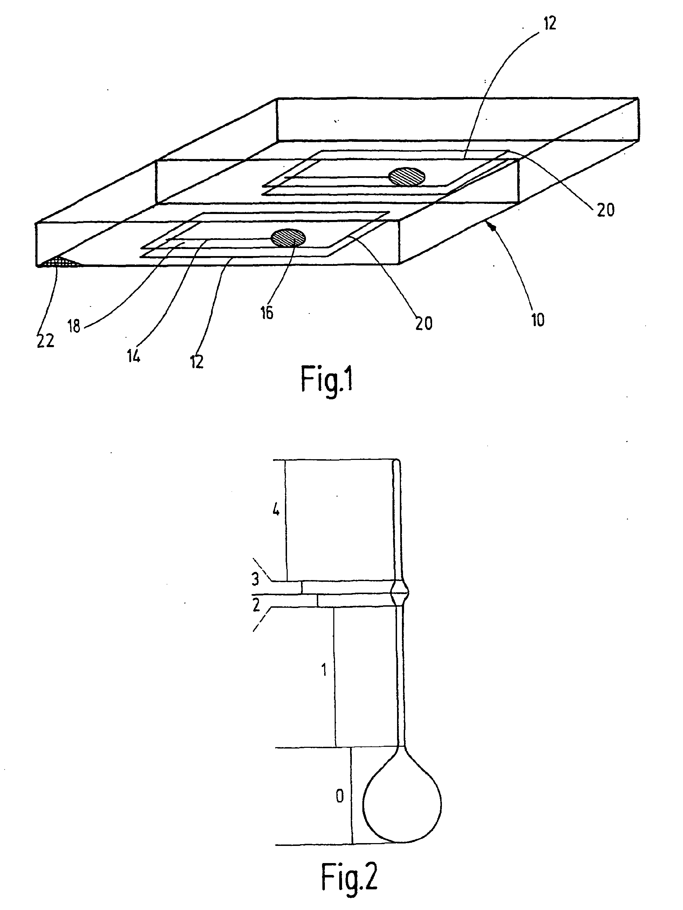

Die in Fig. 1 gezeigte Verpackung 10 kann beispielsweise in Form eines Blistermagazins zur gesonderten Aufnahme einer Mehrzahl von Microsamplern 12 ausgeführt sein. Ein Blistermagazin ist an sich aus der WO 2005/104948 bekannt. Denkbar ist auch die Verwendung in Form eines Trommelmagazins gemäß EP 0951939. Auf die genannten Dokumente wird in diesem Zusammenhang ausdrücklich Bezug genommen.

Der Microsampler oder Mikroprobennehmer 12 ist zur Gewinnung einer kleinen Blutmenge aus einem Körperteil 14 vorgesehen. Er kann aus dünnem Edelstahlblech bestehen, in welchem ein längsoffener rillenförmiger Kapillarkanal 14 von einer distalen Spitze zu einer proximalen Sammelstelle 16 führt, die als Reaktionsbereich für den Nachweis eines Analyten, z. B. Glucose ausgebildet sein kann. Optional kann das gesammelte Blut aber auch in eine nicht gezeigte Analyseeinheit transferiert werden, um dort den Analyten zu bestimmen. Zumindest im Bereich des Kanals 14 und der Sammelstelle 16 ermöglicht eine hydrophile Oberfläche 18 einen verbesserten Flüssigkeitstransport. Hierfür sollte die Hydrophilie der Oberfläche 18 über die vorgesehene Lagerdauer erhalten bleiben, so dass mit deionisiertem Wasser ein Kontaktwinkel kleiner 40° erreicht wird.

Generell sind hydrophile Oberflächen immer auch hochenergetische Oberflächen. Da natürliche Systeme bestrebt sind, ihre Energie zu minimieren, werden hydrophile Oberflächen durch Adsorption von apolaren Gasen oder durch Verschmutzung mit Staub oder andere Kleinstpartikel hydrophobiert. Erfindungsgemäß wird daher die hydrophile Oberfläche 18 durch Lagerung in der Hülle einer geeigneten Verpackung 10 über die Zeit hydrophil erhalten. Zu diesem Zweck ist in der Verpackung 10 eine lose Abdeckung 20 in Form eines Deckplättchens über der mit der hydrophilen Oberfläche 18 versehenen Seite des Microsamplers 12 angeordnet. Die Abdeckung 20 kann eine mechanische Barriere bilden bzw. als vorgelagertes Adsorberelement mit einer hydrophilen Beschichtung versehen sein, deren Hydrophilie gleich oder größer ist als die Hydrophilie der Oberfläche 18. Möglich ist es auch, dass die adsorbierende Oberfläche in Form einer hydrophilen Beschichtung 22 zumindest eines Teils der Innenseite der Verpackung 10 vorgesehen ist, deren Hydrophilie gleich oder größer ist als die Hydrophilie der Oberfläche 18. Der Einfachheit halber ist die Beschichtung 22 in Fig. 1 nur ausschnittsweise veranschaulicht.

Vorteilhafterweise besteht die hydrophile Beschichtung 22 aus mindestens einer linearen oder vernetzten Polyacrylsäure und/oder mindestens einem linearen oder vernetzten Polyacrylat.

Für einen Vergleichsversuch wurden Testplättchen aus Medizinalstahl mit einem Reservoir und einer rillenförmigen Kapillare versehen. Diese Strukturen wurden, wie aus Fig. 2 ersichtlich, in fünf Bereiche gegliedert. Der Bereich 0 umfasst das Reservoir, der Bereich 1 den unteren Teil der Kapillare, die Bereiche 2 und 3 eine Verbreiterung der Kapillare und der Bereich 4 den oberen Teil der Kapillare. Eine Hälfte der Testplättchen wurde mit einem dem Fachmann bekannten Verfahren mit Lecithin und die andere Hälfte der Testplättchen mit Dextransulfat beschichtet. Anschließend wurden die Testplättchen in Mylar-Folie (Hersteller: DuPont) mit einer Dicke von 20 µm verpackt. Dabei wurde jeweils eine Hälfte der mit Lecithin und Dextransulfat beschichteten Plättchen ungeschützt verpackt und die jeweils andere Hälfte vor dem Verpacken mit einem Deckplättchen zum Schutz der Kapillare versehen. Es lagen also vier verschiedene Arten von verpackten Testplättchen vor:
a) mit Lecithin beschichtet, ohne Schutz;
b) mit Lecithin beschichtet, mit Deckplättchen;
c) mit Dextransulfat beschichtet, ohne Schutz;
d) mit Dextransulfat beschichtet, mit Deckplättchen.

Die fertig verpackten Testplättchen wurden mit Elektronenstrahlen (β-Strahlen) sterilisiert (25kGy, 10meV) und während 12 Wochen bei 35°C gelagert. Damit wurde eine Lagerung über 2 Jahre bei Raumtemperatur simuliert.

Jede zweite Woche wurden Plättchen entnommen und getestet. Dazu wurde jeweils 1,5µl Blut in das Reservoir der Kapillaren pipettiert und das Befüllverhalten der Kapillaren anhand der in Fig. 2 dargestellten und oben beschriebenen Einteilung in die Bereiche 0 bis 4 bewertet.

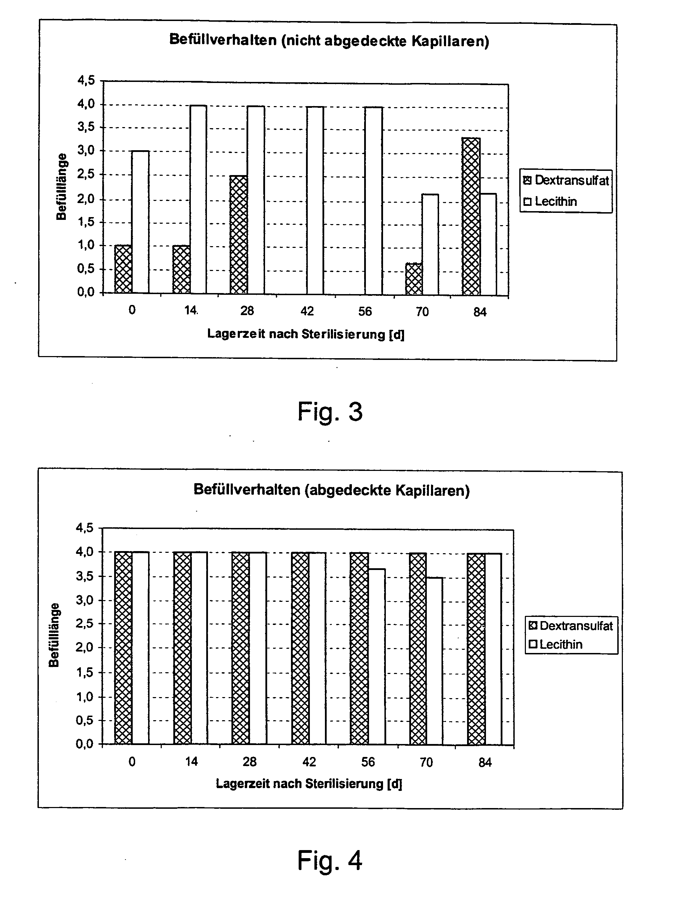

Die Ergebnisse sind in den Figuren 3 und 4 als Säulendiagramme dargestellt. Es ist gut zu erkennen, dass die geschützten Kapillaren auch nach der maximalen Lagerdauer sich noch vollständig füllen, wenn sie mit der Beschichtung aus Dextransulfat versehen sind und sich vollständig oder fast vollständig füllen, wenn sie mit der Beschichtung aus Lecithin versehen sind. Bei den ungeschützten Kapillaren schneiden diejenigen mit der Lecithin-Beschichtung besser ab; das Befüllverhalten ist jedoch insgesamt deutlich schlechter als bei den mit Dextransulfat beschichteten Kapillaren, d.h. die Kapillaren füllen sich nur teilweise oder gar nicht.

## Patentansprüche

1. Verpackung enthaltend ein Mikrofluidikelement (12) mit einer hydrophilen Oberfläche (18) zur Aufnahme einer Körperflüssigkeit und mindestens eine die hydrophile Oberfläche (18) überdeckende lose Abdeckung (20) und/oder mindestens eine adsorbierende Oberfläche (22), wobei die Affinität der Abdeckung und/oder adsorbierenden Oberfläche (22) für apolare Gase gleich groß oder größer ist als diejenige der hydrophilen Oberfläche.

2. Verpackung nach Anspruch 1, **dadurch gekennzeichnet, dass** die lose Abdeckung (20) als Deckplättchen oder Deckvlies ausgebildet ist.

3. Verpackung nach Anspruch 1 oder 2, **dadurch gekennzeichnet, dass** die adsorbierende Oberfläche (22) in Form eines mit dem Mikrofluidikelement (12) verpackten Adsorberelements mit adsorbierender Oberfläche vorgesehen ist.

4. Verpackung nach Anspruch 3, **dadurch gekennzeichnet, dass** das Adsorberelement zugleich als lose Abdeckung (20) für die hydrophile Oberfläche ausgebildet ist.

5. Verpackung nach Anspruch 3 oder 4, **dadurch gekennzeichnet, dass** das Adsorberelement mit einer hydrophilen Beschichtung versehen ist, deren Hydrophilie gleich oder größer ist als die Hydrophilie der Oberfläche.

6. Verpackung nach Anspruch 1, **dadurch gekennzeichnet, dass** die adsorbierende Oberfläche (22) in Form einer hydrophilen Beschichtung zumindest eines Teils der Innenseite der Verpackung vorgesehen ist, deren Hydrophilie gleich oder größer ist als die Hydrophilie der hydrophilen Oberfläche des Mikrofluidikelements (12).

7. Verpackung nach Anspruch 5 oder 6, **dadurch gekennzeichnet dass** die hydrophile Beschichtung mindestens eine lineare oder vernetzte Polyacrylsäure und/oder mindestens ein lineares oder vernetztes Polyacrylat enthält.

8. Verpackung nach Anspruch 5 oder 6, **dadurch gekennzeichnet, dass** die hydrophile Beschichtung aus mindestens einer linearen oder vernetzten Polyacrylsäure und/oder mindestens einem linearen oder viernetzten. Polyacrylat besteht.

9. Verpackung nach einem der Ansprüche 5 bis 8, **dadurch gekennzeichnet, dass** die hydrophile Beschichtung aus dem gleichen Material besteht wie die hydrophile Oberfläche des verpackten Mikrofluidikelements (12).

10. Verpackung nach einem der Ansprüche 1 bis 9, **dadurch gekennzeichnet, dass** die hydrophile Oberfläche (18) so erhalten bleibt, dass sie mit deionisiertem Wasser einen Kontaktwinkel von weniger als 80°, vorzugsweise weniger als 40° bildet.

11. Verpackung nach einem der vorhergehenden Ansprüche in Form eines Magazins für eine Mehrzahl von Mikrofluidikelementen.

## Claims

1. Packaging containing a microfluidic element (12) having a hydrophilic surface (18) for taking up a body fluid and at least one loose cover (20) which covers the hydrophilic surface (18) and/or at least one adsorbing surface (22), wherein the affinity of the cover (20) and/or adsorbing surface (22) for apolar gases is equal to or greater than that of the hydrophilic surface.

2. Packaging according to claim 1, **characterized in that** the loose cover (20) is in the form of a cover plate or cover fleece.

3. Packaging according to claim 1 or 2, **characterized in that** the adsorbing surface (22) is provided in the form of an adsorber element having an adsorbing surface that is packaged with the microfluidic element (12).

4. Packaging according to claim 3, **characterized in that** the adsorber element is at the same time in the form of a loose cover (20) for the hydrophilic surface.

5. Packaging according to claim 3 or 4, **characterized in that** the adsorber element is provided with a hydrophilic coating, the hydrophilicity of which is equal to or greater than the hydrophilicity of the surface.

6. Packaging according to claim 1, **characterized in that** the adsorbing surface (22) is provided in the form of a hydrophilic coating of at least a part of the inside of the packaging, the hydrophilicity of which is equal to or greater than the hydrophilicity of the hydrophilic surface of the microfluidic element (12).

7. Packaging according to claim 5 or 6, **characterized in that** the hydrophilic coating contains at least one linear or cross-linked polyacrylic acid and/or at least one linear or cross-linked polyacrylate.

8. Packaging according to claim 5 or 6, **characterized in that** the hydrophilic coating consists of at least one linear or cross-linked polyacrylic acid and/or at least one linear or cross-linked polyacrylate.

9. Packaging according to one of the claims 5 to 8, **characterized in that** the hydrophilic coating consists of the same material as the hydrophilic surface of the packaged microfluidic element (12).

10. Packaging according to one of the claims 1 to 9, **characterized in that** the hydrophilic surface (18) is maintained in such a manner that it forms a contact angle of less than 80°, preferably less than 40° with deionized water.

11. Packaging according to one of the previous claims in the form of a magazine for a plurality of microfluidic elements.

## Revendications

1. Emballage contenant un élément microfluidique (12) présentant une surface hydrophile (18) destinée à absorber un liquide corporel et au moins un recouvrement (20) lâche recouvrant la surface hydrophile (18) et/ou au moins une surface adsorbante (22), l'affinité du recouvrement et/ou de la surface adsorbante (22) pour les gaz apolaires étant identique ou supérieure à celle de la surface hydrophile.

2. Emballage selon la revendication 1, **caractérisé en ce que** le recouvrement lâche (20) est réalisé sous forme de plaquette ou de non-tissé de recouvrement.

3. Emballage selon la revendication 1 ou 2, **caractérisé en ce que** la surface adsorbante (22) est prévue sous forme d'un élément adsorbant avec une surface adsorbante emballé avec l'élément microfluidique (12).

4. Emballage selon la revendication 3, **caractérisé en ce que** l'élément adsorbant est réalisé simultanément comme recouvrement (20) lâche pour la surface hydrophile.

5. Emballage selon la revendication 3 ou 4, **caractérisé en ce que** l'élément adsorbant est pourvu d'un revêtement hydrophile, dont l'hydrophilie est identique ou supérieure à l'hydrophilie de la surface.

6. Emballage selon la revendication 1, **caractérisé en ce que** la surface adsorbante (22) est prévue sous forme d'un revêtement hydrophile d'au moins une partie de la face interne de l'emballage, dont l'hydrophilie est identique ou supérieure à l'hydrophilie de la surface hydrophile de l'élément microfluidique (12).

7. Emballage selon la revendication 5 ou 6, **caractérisé en ce que** le revêtement hydrophile contient au moins un poly(acide acrylique) linéaire ou réticulé et/ou au moins un polyacrylate linéaire ou réticulé.

8. Emballage selon la revendication 5 ou 6, **caractérisé en ce que** le revêtement hydrophile est constitué par au moins un poly(acide acrylique) linéaire ou réticulé et/ou au moins un polyacrylate linéaire ou réticulé.

9. Emballage selon l'une quelconque des revendications 5 à 8, **caractérisé en ce que** le revêtement hydrophile est constitué par le même matériau que la surface hydrophile de l'élément microfluidique emballé (12).

10. Emballage selon l'une quelconque des revendications 1 à 9, **caractérisé en ce que** la surface hydrophile (18) est conservée de manière telle qu'elle forme un angle de contact avec l'eau désionisée inférieur à 80°, de préférence inférieur à 40°.

11. Emballage selon l'une quelconque des revendications précédentes sous forme d'un chargeur pour une multitude d'élément microfluidiques.
